# EUROPEAN PATENT APPLICATION

(11) **EP 3 771 687 A1**
(43) Date of publication of application: **03.02.2021**
(21) Application number: 19189065.6
(22) Date of filing: 30.07.2019
(51) Int. Cl.: B82Y 5/00, C07K 14/435, G01N 33/487

(54) **REVERSIBLE PHOTO-CONTROLLED FRAC NANOPORES, MODIFIED FRAC MONOMERS, METHODS FOR PREPARING THE SAME, AND USES THEREOF**

(71) Applicant: RIJKSUNIVERSITEIT GRONINGEN, 9712 CP Groningen (NL); Academisch Ziekenhuis Groningen, 9713 GZ Groningen (NL)
(72) Inventor: Maglia, Giovanni, 9747 AG Groningen (NL); Mutter, Natalie Lisa, 9747 AG Groningen (NL); Volaric, Jana, 9747 AG Groningen (NL); Feringa, Bernard Lucas, 9747 AG Groningen (NL); Szymanski, Wiktor Czeslaw, 9713 GZ Groningen (NL)
(74) Representative: V.O.

(57) **Abstract**

The invention relates to the fields of photopharmacology and photo-controlled analyte sensing. More in particular, it relates to a pore forming toxin Fragaceatoxin C (FraC) from *Actina fragacea* that can be reversibly controlled by using light as external trigger. Provided is a modified FraC monomer capable of forming a photo-controlled FraC nanopore, the FraC monomer comprising a photoswitchable moiety, e.g. an azobenzene-based photoswitch, covalently attached to one or more of the positions K77, W112, E134, Y138, and S166.

## Description

The invention relates to the fields of photopharmacology and photo-controlled analyte sensing. More in particular, it relates to a pore forming toxin FraC from *Actina fragacea* that can be reversibly controlled using light as external trigger.

Biological nanopores are proteins that open nanoscale water conduits on biological or synthetic membranes. Under an external potential, the ionic current across single nanopore is suitably used to recognize analytes traversing the nanopore. Most notably, nanopores are now used to sequence nucleic acids at the single-molecule level. In nanopore DNA sequencing, individual nucleic acid chains are threaded base-by-base through nanopores, while the ionic current is used to identify individual nucleobases.

Fragaceatoxin C (FraC) nanopore from the sea anemone *Actinia fragacea* is a pore forming toxin that is expressed as water-soluble monomer and self-assembles into (octameric) transmembrane pores. WO2018/012963 in the name of the applicant shows that FraC nanopores can be used to study DNA, proteins and peptides. In a subsequent study (Huang et al. Nature Commun. (2019) Feb 19), it was surprisingly found that the FraC nanopore can be engineered to induce the formation of different nanopore types when comprised in the context of a lipid bilayer, thereby creating a biological nanopore with sub-nm constriction. Importantly, these novel, narrow types of nanopores allow for distinguishing (small) peptides differing by the substitution of one amino acid with a ∼40 Da resolution. Endogenous protein channels are also important pharmaceutical targets.¹ Different approaches have been attempted to control channels with incorporating photoswitches.²⁻⁵ Light-sensitive soluble small molecules were shown to act as inhibitors causing clogging or closing of the pore.⁶⁻¹¹

The present inventors recognized the potential of FraC nanopores to be used as therapeutic agent based on its capacity to induce cell death. However, a major challenge in pharmacology, in particularly for the design of cancer drugs that rely on cell killing, is reducing off-target toxicity to prevent side-effects.

Therefore, they set out to develop a FraC-based pore-forming toxin that allows for *in situ* reversible activation of the toxin. Ideally, the activity of the toxin can be controlled in a reversible manner with the application of visible light. Preferably, activity of the FraC toxin is activated upon exposure to light.

It was surprisingly found that these goals were met by the provision of a FraC monomer that is modified with a photoswitchable moiety at one or more selected sites to afford a mutant FraC capable of forming a photo-controlled FraC nanopore. More in particular, different azobenzene pendants capable of undergoing *trans-cis* photoisomerization were covalently attached to a set of cysteine-modified FraC monomers *via* a reactive chloroacetyl moiety and tested for hemolytic activity. It was surprisingly found that modification at position77 or 166 resulted in a higher activity in *trans,* whereas modification at position 134, 112 or 138 with the photoswitch resulted in faster hemolytic activity for the *cis* compared to the *trans* isoform. Importantly, activated toxins could be deactivated in the presence of red blood cells by *in situ* irradiation with white light, indicating that the cytotoxicity could be reversibly controlled by light. To test its usefulness as a chemotherapeutic agent, cytotoxicity of modified FraC was also tested towards human cancer cells. The addition of up to 1.1 µM *trans*-modified FraC to A431 epidermoid carcinoma cells did not cause any cell death, while an equal concentration of *cis* showed cytotoxicity. Furthermore, at the concentration in which the *cis* isomer showed complete killing of A431 cancer cells, the *trans* isomer did not show any hemolytic activity towards healthy red blood cells, pointing towards the usefulness of the FraC variant in photoactivated cancer therapy.

Accordingly, the invention provides a modified Fragaceatoxin C (FraC) monomer capable of forming a photo-controlled FraC nanopore, the modified FraC monomer comprising a photoswitchable moiety covalently attached to one or more of the following positions K77, W112, E134, Y138 and S166. Whereas FraC monomers comprising photoswitchable moieties at multiple modification positions are encompassed, the invention is most easily practiced using FraC monomers that are modified only at a single modification site. Hence, in one embodiment the modified FraC monomer comprises one photoswitchable moiety covalently attached to position K77, W112, E134, Y138 or S166.

As used herein, the numbering of the position(s) to be modified with a photoswitch refers to the position in the sequence of the full length wild-type FraC polypeptide from *Actina fragacea* consisting of 179 amino acids, which is available under the accession number FM958450 in GenBank. Accordingly, it is meant that the photoswitchable moiety is covalently attached to one or more of the positions corresponding to K77, W112, E134, Y138, and S166 in the sequence of FraC from *Actina fragacea.* However, it is to be understood that the modified FraC monomer may contain less or more amino acids than that of wild-type FraC. For example, protein tags that aid in isolation and/or purification of the protein upon expression in a host cell can be added. Such tags are well known in the art. In one aspect, the FraC monomer is provided with a C-terminal His (His6) tag. Also, in addition to the residues that are optionally introduced to facilitate FraC modification with a photoswitch (see further herein below), the FraC mutant may contain mutation(s) that have beneficial effects e.g. on recombinant expression in a (bacterial) host cell. For example, in addition to modifications (substitutions) at one or more of the "photoswitch" positions K77, E134, Y138 and S166, the mutation W112S can be introduced to increase FraC expression in *E. coli* or other types of host cells.

The approach underlying the present invention is not taught or suggested in the art. Attempts to photoregulate pore forming toxins are very limited and to the best of our knowledge in the only example, α-hemolysin (aHL) PFT was modified by adding a photoremovable 2-nitrobenzyl group to cysteine residue previously introduced into a region that was known to trigger hemolysis (Chang 1995). Irradiation with light removed the protecting group and irreversibly activated the toxin towards red blood cells. However, once activated, the protein was toxic towards all cells.

Mutter et al.(ACS Chem Biol. (2018) Oct 2) reported a method that uses a directed evolution approach to prepare pore-forming immunotoxins with caged activity. The design contains three separate elements: a water-soluble pore-forming toxin, including those based on FraC, that oligomerizes on lipid membranes, a membrane targeting unit, and a protein trigger for in *situ* activation by a protease. In contrast to the present invention, activation is irreversible and once activated the pore is toxic towards all cells. Furthermore, the prior art method does not rely on exposure to light.

In one aspect, the invention provides a modified FraC monomer capable of forming a FraC nanopore that is activated upon irradiation, the modified FraC monomer comprising a photoswitchable moiety covalently attached to the monomer at one or more of the positions W112, E134 and Y138, preferably Y138.

In another embodiment, the invention provides a modified FraC monomer capable of forming a FraC nanopore that is deactivated upon irradition, the modified FraC monomer comprising a photoswitchable moiety covalently attached at position K77 and/or S166.

In a preferred aspect, the mutant FraC monomer of the present invention is modified with a photoswitchable moiety covalently attached to one of the positions W112, E134 and Y138. Generally speaking, protein modification is desired at a single site to maintain homogeneity and to minimise loss of function. Whereas protein modification can be achieved by targeting some natural amino acid side chains, this often leads to ill-defined and randomly modified proteins. To facilitate FraC modification with a photoswitch, the original amino acid(s) at positions 77, 112, 134, 138 and/or 166 may be converted to a different amino acid that is more suitable for selective chemical modification with a non-protein moiety.

In one embodiment, the photoswitchable moiety is covalently attached to the monomer after the introduction e.g. by genetic substitution, of a natural amino acid, unnatural amino acid or amino acid analog at one or more of the positions K77, W112, E134, Y138 and S166 that allows for site-specific modification.

Amongst the natural amino acids, it is well known that a cysteine residue combines advantageous properties contributing to its suitability for site-selective modification. These include a unique nucleophilicity and a low natural abundance, both allowing chemo- and regioselectivity.

For example, cysteine (Cys) residues are highly amenable for site-specific chemical protein modification. The wild-type FraC polypeptide sequence does not contain any Cys residues. Hence, introduction of a Cys residue at one or more desired site(s) in the FraC polypeptide by means of reliable genetic engineering techniques is very suitable to provide a modified FraC monomer.

Accordingly, in a preferred embodiment the invention provides a modified FraC monomer wherein a photoswitchable moiety is attached to a mutant FraC wherein a cysteine residue is introduced at one or more of the positions K77, W112, E134, Y138, and S166. For example, the mutant contains one of the mutations K77C, W112C, E134C, Y138C or S166C. In a specific aspect, the mutant FraC comprises the mutation W112C, E134C and/or Y138C. Very good results can be obtained with mutant Y138C.

For example, said one or more photoswitchable moiety is attached to a Cys-residue introduced at one or more of the positions W112, E134 and Y138, thereby resulting in a modified monomer comprising the mutations W112C, E134C and/or Y138C. The invention therefore provides a modified mutant Fragaceatoxin C (FraC) monomer capable of forming a photo-controlled FraC nanopore, the mutant FraC monomer comprising a Cys-residue at one or more of the positions W112, E134 and Y138, and wherein said one or more Cys-residue is covalently modified with a photoswitchable moiety. Preferably, the modified mutant FraC monomer comprises a Cys-residue at one the positions W112, E134 and Y138, and wherein said Cys-residue is covalently modified with a photoswitchable moiety. Hence, the modified mutant may but does not need to contain multiple sites that are modified with a photoswitch.

In one embodiment, the mutant FraC comprises the mutation W112C. Alternatively or additionally, the mutant FraC comprises the mutation E134C. Very good results were obtained with a modified FraC mutant comprising at least the point mutation Y138C. For example, an azobenzene-modified FraC-Y138C mutant showed no hemolytic activity in the resting *trans* isoform, while irradiation with light induced complete red blood cell lysis. Therefore, in yet an alternative or additional embodiment, the mutant FraC comprises the mutation Y138C.

The invention also provides a mutant FraC polypeptide comprising a natural or unnatural amino acid residue introduced at position K77, W112, E134, Y138 and/or S166. In one embodiment, the mutant FraC comprises a Cys-residue at position K77, W112, E134 Y138 and/or S166. Preferably, the invention provides a FraC mutant selected from the group consisting of K77C, W112C, E134C, Y138C and S166C. As indicated above, the mutant may contain additional mutation(s) to aid in expression, purification, isolation etc. In a specific aspect, the mutant contains the mutation W112S in addition to one or more of K77C, E134C, Y138C and S166C.

Also provided is a nucleic acid molecule encoding a mutant FraC polypeptide according to the invention, as well as an expression vector comprising the nucleic acid molecule. Such vector is suitably introduced in a host cell, e.g. a bacterial host cell, for recombinant production of a mutant FraC polypeptide.

As used herein, a photoswitchable molecule or "photoswitch" is a chemical moiety that is sensitive in some way to light. Some examples include azobenzenes, stilbenes, spiropyranes, diarylethenes, fulgides, hemithioindigos and overcrowded alkenes. For instance, azo compounds can isomerize by absorbing light of a particular wavelength. This photoisomerisation transforms the azo group from the *trans* to the *cis* form. In the field of photopharmacology, photoswitches are used to obtain control over the activity. By attaching a photoswitch to drugs, a drug with several states is created, all having their own biological activity. Light can be used to switch between these states, resulting in drugs with remote control over the activity. See for example Velema et al. (2014). "Photopharmacology: Beyond Proof of Principle". J. Am. Chem. Soc. 136 (6): 2178-2191.

A person skilled in the art will understand and appreciate that the concept underlying the present invention resides in the identification of the FraC site(s) to be modified with a photoswitchable moiety, and that various types of photoswitches can be used to put the invention into practice.

Suitable photoswitches for FraC modification are known in the art, and include among others azobenzene-, stilbene- and spiropyrane-based photoswitches.

In one embodiment, the invention is practiced using FraC modified with a spiropyrane-based photoswitch, for example selected from the following compounds:

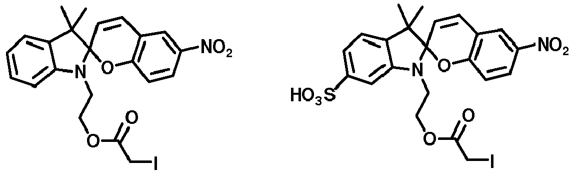

Spiropyrane photoswitches and methods for their synthesis are known in the art. For example, Inoue, M. et al., J. Org. Chem., 1992, 57, 5377-5383 and A. Kocer, M. Walko, B. L. Feringa, Nat. Prot., 2007, 2 (6),1426 describe the synthesis of spiropyrane. Gao et al., Journal of Molecular Structure., 2016, 1123, 426-432) describes the synthesis of sulfonated spiropyrane.

Very good results were obtained when FraC monomers were modified using azobenzene-based photoswitches , in particular those comprises at the *para-*position a negatively or positively charged substituent to increase its water solubility. In a preferred aspect, the azobenzene-based photoswitch comprises a sulfonate group or a quaternary ammonium group at the *para-*position.

Azobenzene-based photoswitches can suitably be attached via a reactive chloroacetyl moiety (e.g. at the para-position) to a cysteine-residue introduced at one or more of the positions K77, W112, E134, Y138 and S166.

In one embodiment, the photoswitchable moiety is reversibly responsive to UV and/or visible light. In a preferred embodiment, the photoswitchable moiety is reversibly responsive to visible light. For example, the photoswitch comprises an azobenzene core that is modified to induce a red shift in the absorption spectrum of the molecule, allowing reversible switching using irradiation with blue (500 nm) and green (410 nm) light. The inventors surprisingly succeeded to synthesize a visible light-controlled azobenzene by introducing four fluorine moieties in the *ortho*-positions. This *ortho*-modified core preferably further comprises a water-solubilizing group, e.g. sulfonate, at the *para*-position.

Accordingly, the invention also relates to a visible light-controlled azobenzene-based photoswitch and the synthesis thereof. In one embodiment, the invention provides a compound of the formula I 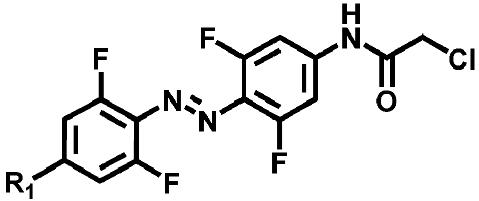 wherein R₁ is a charged group conferring water-solubility. In one embodiment, R₁ is a negatively charged group e.g., carboxy, sulfonate, phosphate or phosphonate. By "sulfonate" is meant sulfonic acid, or salts of sulfonic acid (sulfonate). Similarly, by "carboxy" is meant carboxylic acid or salts of carboxylic acid. "Phosphate", as used herein, is an ester of phosphoric acid, and includes salts of phosphate., In another embodiment, R₁ is a positively charged group e.g. (quaternary) ammonium.

In a further embodiment, the invention provides a method for the manufacture of a visible light responsive photoswitch compound of formula I. The synthetic method involves an electron-poor azobenzene molecule, such as the azobenzene containing 4 fluorines in the *ortho* positions, having a fluoro group in the *para* position. The fluoride is prone to react with a nucleophile, such as sulfite anion, in a nucleophilic aromatic substitution reaction providing installation of the water solubilizing sulfonate group. On the other *para* position the molecule has a bromo group which does not participate in the nucleophilic aromatic substitution reaction leaving it intact during sulfonate incorporation. Afterwards, the bromo group provides the possibility of functionalization of the water-soluble azobenzene via Buchwald-Hartwig coupling to install the amide.

An exemplary embodiment of this method is depicted in the following scheme:

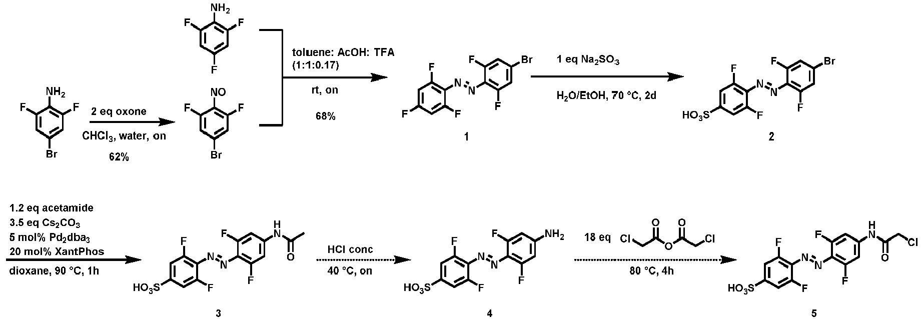

A further aspect of the invention relates to a photo-controlled FraC nanopore comprising a modified FraC monomer as herein disclosed. Depending on the site of modification of the FraC monomer, the nanopore is either activated or inactivated upon irradiation. In one embodiment, the invention provides a FraC nanopore that is activated upon irradiation, which nanopore comprises a modified FraC monomer comprising a photoswitchable moiety covalently attached to one or more of the positions W112, E134 and Y138. In a specific aspect, the invention provides a FraC nanopore that is activated upon irradiation, which nanopore comprises a modified FraC monomer comprising a photoswitchable moiety covalently attached to position Y138, preferably to a mutant FraC wherein a cysteine is introduced at position W112 (mutant W112C), E134 (mutant E134C) or Y138 (mutant Y138C).

In another specific aspect, the invention provides a FraC nanopore that is inactivated upon irradiation, comprising a mutant FraC monomer wherein a photoswitchable moiety covalently attached to position K77 and/or S166, preferably to a mutant FraC wherein a cysteine is introduced at position K77 (mutant K77C) or S166 (mutant S166C).

Also provided is a method for providing a photo-controlled FraC nanopore according to the invention, comprising providing a composition comprising modified FraC monomers according to the invention and exposing the composition to light capable of inducing a conformational change in the photoswitch, thereby controlling assembly of a FraC nanopore. For example, the method comprises providing a modified (mutant) FraC monomer as herein disclosed, by reacting a (mutant) FraC with a photoswitchable compound under conditions that allow for covalent modification, and separating the modified FraC (mutant) from unconjugated FraC monomers. The modified FraC monomer can then be exposed to light capable of inducing a conformational change in the photoswitch, thereby controlling assembly of a FraC nanopore. In a specific aspect, a Cys-containing FraC mutant is reacted with a photoswitchable compound on a chromatography column, in the dark.

The photo-controlled FraC nanopores of the invention have several interesting applications. For example, for analytical applications, the nanopore can be included in a system wherein the nanopore is assembled in a (planar) lipid bilayer. The lipid bilayer may comprise or consist of phosphatidylcholine (PC), preferably 1,2-diphytanoyl-*sn*-glycero-3-phosphocholine.

A photo-controlled FraC nanopore or system according to the invention is advantageously used as (bio)molecule sensor, preferably as a single-molecule sensor. Also provided is a sensing device comprising a modified FraC monomer, a photo-controlled FraC nanopore or a system as herein disclosed. The sensing device is for example an electrophysiology chamber comprising two compartments separated by a film that contains a small (e.g. 50-200 µm diameter) orifice which contains a lipid bilayer comprising a photo-controlled FraC nanopore.

For example, it was found that the *trans* isomer of a modified FraC monomer could not form nanopores, while direct irradiation of the electrophysiology chamber with 365 nm light, forming the *cis* isomer, activated the nanopores as shown by stepwise increases of the bilayer conductance reflecting individual insertions into the planar lipid bilayer. Importantly, subsequent *in situ* irradiation with white light almost completely inhibited further assembly of FraC nanopores. Thus, once a single nanopore is inserted, the excess of nanopores in solution can be removed by irradiation with white light, thereby greatly reducing the probability of a second insertion. This property greatly facilitates precise preparation of arrays of single nanopores, allow among others to reduce the cost of multiplexing. Accordingly, a further embodiment relates to the use of a photocontrolled FraC-based sensing device in multiplexing technology.

Other important aspects of the invention relate to photopharmacological and medical (therapeutic) applications. As described herein above, a photo-controlled FraC nanopore as provided herein allows for the reversible regulation of its toxic activity towards biological membranes using light. The cytotoxicity of a photo-activatable FraC nanopore towards human cancer cells was shown, indicating its usefulness as a chemotherapeutic agent. Thus, in one embodiment the invention provides a photo-controlled FraC nanopore according to the invention for use as medicament, preferably as cytotoxic agent, in particular as chemotherapeutic agent. Also provided is a pharmaceutical composition comprising a modified FraC monomer and a pharmaceutically acceptable carrier, vehicle or diluent. The pharmaceutical composition preferably comprises a modified (mutant) FraC monomer capable of forming / assembling into a light-activated FraC nanopore upon irradiation, in particular a mutant FraC monomer comprising a photoswitchable moiety covalently attached to one or more of the positions W112, E134 and Y138, preferably Y138. More preferably, a modified FraC monomer for therapeutical or pharmaceutical use is modified with a visible-light responsive photoswitch to allow for *in situ* irradiation within a living organism.

Also provided is a photo-controlled FraC nanopore or a pharmaceutical composition according to the invention for use in a method for photo-controlled cytotoxic therapy in a subject, for example in the treatment of cancer.

In one embodiment, a method for photo-controlled cytotoxic therapy in a subject comprises administering to said subject a pharmaceutical composition of the invention, and exposing at least a body part of the subject that is known or assumed to contain the modified FraC monomer to a suitable wavelength of light to induce FraC nanopore formation. Preferably, the subject is human.

### LEGEND TO THE FIGURES

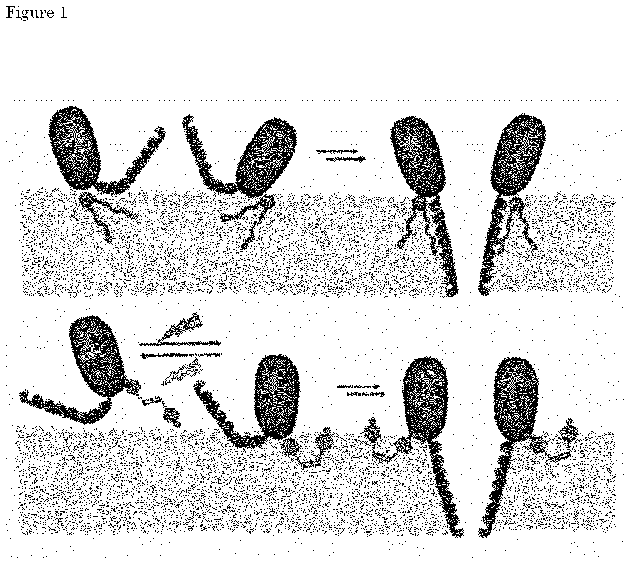

Figure 1 : Cartoon representation of photo-controlled assembly of FraC nanopore. Top) Mechanism of FraC (purple) pore formation where sphingomyelin (red) present in the lipid bilayer (yellow) stabilizes the formed pore. Bottom) FraC modified with a photoswitch (red) does not bind to the surface of the cell when the azobenzene is in the *trans* state, possibly because the charged group in para-position limits the affinity with the hydrophobic bilayer. The azobenzene switch can be reversibly switched to the *cis* state upon irradiation with light, allowing binding to the surface of the cell and forming a pore. The mature pore structure is stabilized by the cis-azobenzene.

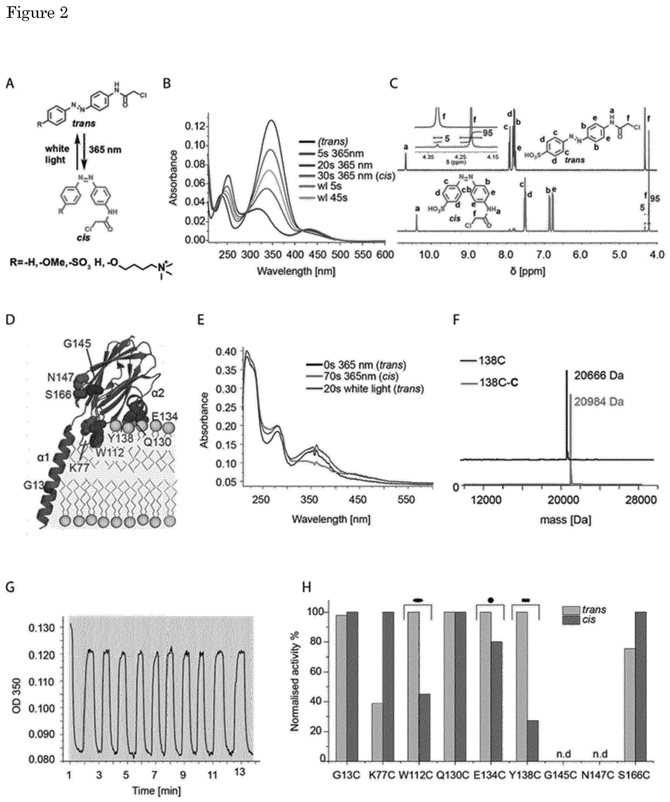

Figure 2. Engineering a light-activated toxin. A) Chemical structures of the azobenzene switch molecules and their activation with light. B) UV-Vis spectra of switch C (20 µM, 25 °C) before, during irradiation at 365 nm, and after reaching the PSS state as well as back-switching with white light. Measurement were done in 150 mM NaCl, 15 mM Tris · HCl pH 7.5. C) 1H-NMR spectra of C (6 mM, 25 °C) in DMSO-d6 before (trans, purple spectrum) and after irradiation at 365 nm (cis, red spectrum). D) Cartoon representation of FraC (purple, PDB: 4TSY55) bound to lipids (yellow)) showing the amino acids substituted with cysteine (blue spheres), which were then modified by C. E) UV-Vis spectra of FraC-Y138C-C (15 µM, 25 °C) before irradiation, during irradiation at 365 nm and after irradiation with white light. F) ESI-MS spectra of FraC 138C and FraC-Y138C-C. G) Reversible photoswitching of FraC-Y138C-C (15 µM, 25 °C) followed by UV-Vis spectroscopy. H) Hemolytic activity of FraC-C in the cis and trans confor-mations. The bars show the normalized t50 for the different FraC mutant attached to switch C in cis and trans state. The bar, dot and ellipse indicate mutants with faster hemolytic activity in cis state.

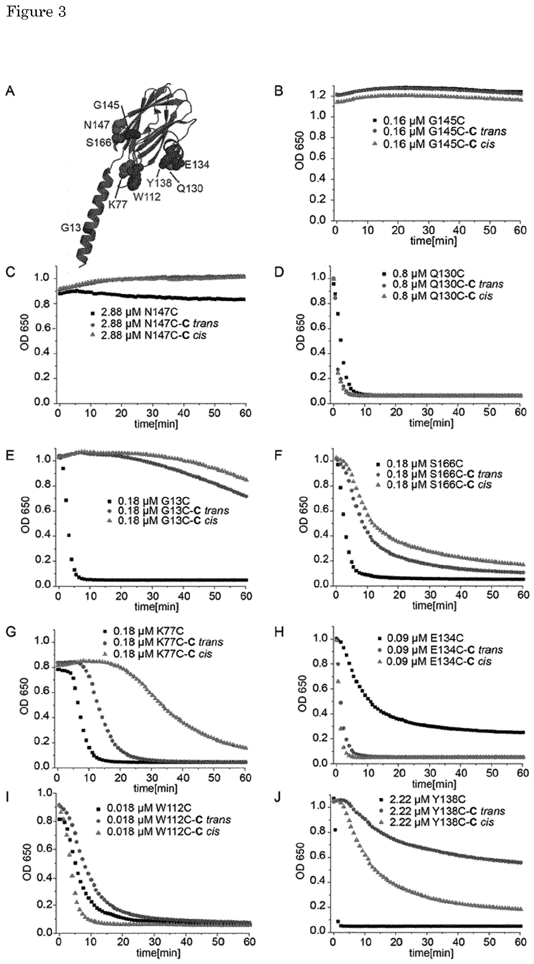

Figure 3. Hemolytic activity of FraC-C constructs. A) Schematic representation of FraC (purple, PDB: 4TSY) bound to lipids (yellow). Amino acid residues G13, K77, Q130, E134, Y138 G145, N147 and S166 (highlighted as blue spheres) were mutated into cysteine in order to attach an azobenzene at different positions at the membrane interface. B to I) Hemolytic activity of FraC constructs with a substitute cysteine residue at the indicated position (black squares), the same construct modified with switch C (purple spheres), and after irradiation at 365 nm (red triangles). B and C) The introduction of a cysteine residue at position N147 and G145 reduced dramatically the hemolytic activity and attachment of the azobenzene resulted in an inactive toxin. Irradiation with UV light could not restore hemolytic activity. Both residues are at the interface between protomers, most likely important for oligomerization of FraC. D) Modification of cysteine residue Q130 did not change hemolytic activity, all variants lysed blood rapidly, assuming no direct interaction with the membrane. E) Modification with switch C at position G13 decreased hemolytic activity drastically due to steric hindrance between the protomers. Isomerization of the azobenzene by UV light (365 nm) did not increase the hemolytic activity. F and G) Modification with switch C at position K77 and S166 decreased hemolytic activity. The *trans* configuration was more active than the *cis* configuration. H) Position E134 is the only mutant with increased hemolytic activity after modification, due to reintroducing a negative charge while attaching switch C. Isomerization of the azobenzene from *trans* to *cis* by UV light (365 nm) resulted in a small increase of hemolytic activity. I-J) Modification with switch C at position W112 and Y138 decreased hemolytic activity. Isomerization of the azobenzene from *trans* to *cis* by UV light (365 nm) partially restored the hemolytic activity. All proteins were purified by Ni-NTA affinity chromatography and not further separated from unmodified C-FraC.

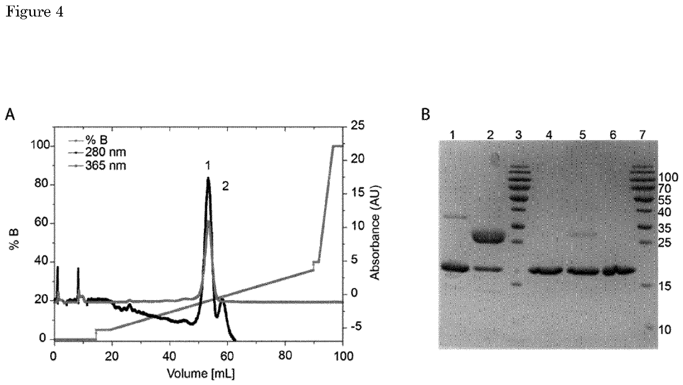

Figure 4. Purification of FraC-Y138C-C. A) Chromatogram obtained from eluting FraC-Y138C-C from a cation exchange column (HiTrap SP HP column). Peak 1 corresponds to modified FraC and peak 2 to unmodified FraC monomers. B) Modification of Y138C FraC with switch C examined by 12% SDS-PAGE electrophoresis. Lane 1:Y138C FraC. Lane 2: Y138C FraC modified with PEG-5000-maleimide. Lane 3: protein ladder. Lane 4: FraC-Y138C-C after His-Tag purification. Lane 5: FraC-Y138C-C after His-Tag purification and incubated with PEG-5000-maleimide. Lane 6: concentrated peak 1 from cation exchange chromatography containing FraC-Y138C-C. Lane 7: protein ladder.

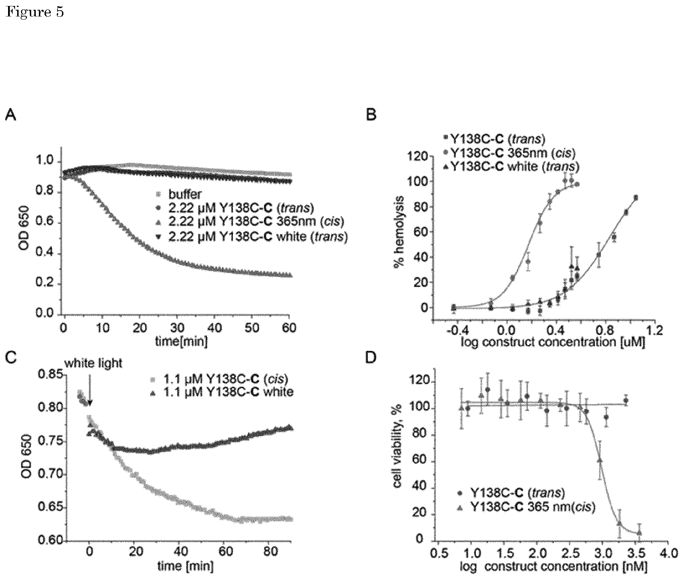

Figure 5. A light-activated FraC nanopore toxin. A) In the *trans* state (no irradiation, purple spheres) FraC-Y138C-C is not hemolytic active. The conversion to the *cis* state (irradiation at 365 nm, red triangles) triggers the hemolytic activity of the nanopore. Nanopore solutions first activated by irradiation at 365 nm and then deactivated with white light (blue triangles) are not hemolytically active. B) HC50 of FraC-Y138C-C variants in the *cis* form (irradiated with λ=365 nm, pink squares) *trans* form (no irradiation, purple square and irradiation with white light, blue triangles). C) Comparison of the hemolysis percentage of FraC-Y138C-C in the *trans* state (no irradiation, purple square and irradiation with white light, blue triangles), and in the *cis* state (irradiation at 365 nm, red spheres).). D) Toxicity of FraC-Y138C-C towards A431 cancer cells. Activated FraC-Y138C-C (irradiation at 365 nm, red triangle) is cytotoxic, while deactivated FraC-Y138C-C (irradiated with white light, purple spheres) does not kill the cells.

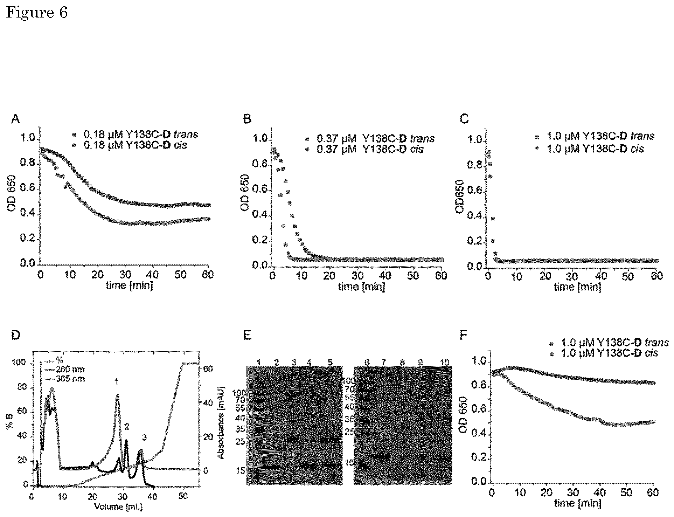

Figure 6. Hemolytic activity and purification of FraC-Y138C-D. A to C) Hemolytic activity of FraC-Y138C modified with switch D (purple squares), and after irradiation at 365 nm (red spheres) at different concentrations. Faster hemolytic activity of FraC-Y138C-D compared to FraC-Y138C-C was observed, because switch D modification was less efficient and more unmodified FraC was present. D) Chromatogram obtained from eluting FraC-Y138C-D from a cation exchange column (HiTrap SP HP column). Peak 1 corresponds to free azobenzene, peak 2 to unmodified FraC and peak 3 to modified FraC monomers. E) Modification of Y138C FraC with D examined by 12% SDS-PAGE electrophoresis. Lane 1: protein ladder. Lane 2:Y138C FraC. Lane 3: Y138C FraC modified with PEG-5000-maleimide. Lane 4: FraC-Y138C-D after His-Tag purification. Lane 5: FraC-Y138C- D after His-Tag purification and incubated with PEG-5000-maleimide. Lane 6: protein ladder. Lane 7: concentrated peak 3 from cation exchange chromatography containing FraC-Y138C-D. Lane 8: the flow through from cation exchange chromatography containing no FraC. Lane 9: peak 1 from cation exchange chromatography containing little FraC. Lane 10: peak 2 from cation exchange chromatography containing unmodified FraC. F) Hemolytic activity of cation exchange chromatography purified FraC-Y138C-D in the *trans* configuration (no irradiation, purple spheres), in the *cis* configuration (upon irradiation at 365 nm, red triangles).

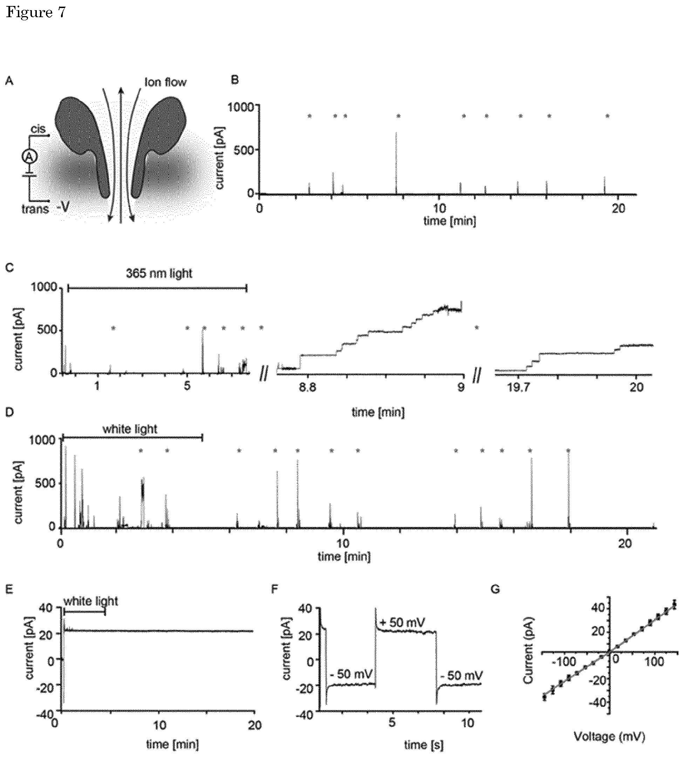

Figure 7. A light-activated nanopore. A) Schematic representation of nanopore experiment showing a FraC nanopore (purple) inserted in a planar lipid bilayer (blue). B) The addition of 4 µM of FraC-Y138C-C (*trans* conformation) to the *cis* side of an electrophysiology chamber (500 µL) did not induce the formation of nanopores, as shown by the lack of changes of the bilayer conductance. Every few minutes the bilayer was reformed (red asterisk) to ensure a stable bilayer formation. C) About ten minutes after irradiating the electrophysiology chamber with UV light at λ = 365 nm, stepwise insertions of several nanopores are observed as detected by discrete current enhancements. D) The subsequent irradiation of the *cis* chamber (5 min, white light) prevented the insertion of additional nanopores. E) After a single FraC-Y138C-C was reconstituted, the insertion of additional nanopores was prevented by irradiated with white light. F and G) Relationship between current and voltage for single FraC-Y138C-C nanopores. The solution used for the electrical recording contained 1 M NaCl, 15 mM Tris HCl, pH 7.5. Current traces from B to E were collected applying +50 mV, a Bessel low/pass filter with 2 kHz cutoff and sampled at 10 kHz at room temperature (25 °C).

### EXPERIMENTAL SECTION

### General information

All chemicals for synthesis were obtained from commercial sources and used as received unless stated otherwise. DNA was purchased from Integrated DNA Technologies (IDT). Enzymes were acquired from Fermentas and lipids from Avanti Polar Lipids. Technical grade solvents were used for extraction and chromatography. Thin Layer Chromatography (TLC) was performed using commercial Kiesegel 60 F254 silica gel plates with fluorescence-indicator UV254 (Merck, TLC silica gel 60 F254) for normal phase and Nano-Silica gel RP-18W on Alu-foil (Sigma Aldrich) for reversed phase. For detection of components, UV light at λ=254 nm or λ=365 nm was used. Alternatively, oxidative staining was performed using a basic solution of potassium permanganate in water or aqueous cerium phosphomolybdic acid solution (Seebach's stain). Merck silica gel 60 (230-400 mesh ASTM) was used in normal phase flash chromatography. Reveleris® X2 automatic column was used with Reveleris® C18 40 µm columns for reversed phase purification. Preparative HPLC purification was performed on a Shimadzu HPLC system with a Phenomenex® Kinetex 5 µm EVO C18 100Å column.

Spectroscopic measurements were made in Uvasol® grade solvents using a quartz cuvette (path length 10.00 mm). UV-Vis measurements were performed on an Agilent 8453 UV-Visible absorption Spectrophotometer. For biological experiments a Synergy H1 plate-reader was used (BioTek). UV-Vis irradiation experiments were carried out using a 365 nm LED (Thorlabs M365F1) and a white light source (Thorlabs Fiber Illuminator OSL1-EC).

NMR spectra were obtained using Agilent Technologies 400-MR (400/54 Premium Shielded) (400 MHz) and Bruker Innova (1H: 600 MHz, 13C: 151 MHz) spectrometers at room temperature (22-24 °C). Chemical shift values (δ) are reported in parts per million (ppm) with the solvent resonance as the internal standard (CDCl₃: δ 7.26 for ¹H, δ 77.16 for ¹³C; DMSO: δ 2.05 for ¹H, δ 39.52 for 13C). The following abbreviations are used to indicate signal multiplicity: s (singlet), d (doublet), t (triplet), q (quartet), m (multiplet), brs (broad signal) or dd (doublet of doublets).

Exact mass spectra were recorded on an LTQ Orbitrap XL (ESI+). All reactions requiring an inert atmosphere were carried out under a nitrogen atmosphere using oven dried glassware and standard Schlenk techniques. Dichloromethane and toluene were used from solvent purification system using an MBraun SPS-800 column. Melting points were determined using Stuart analogue capillary melting point SMP₁₁ apparatus. All errors are given as standard deviations.

### Chemical Synthesis of UV-responsive Photoswitches

### Switch A

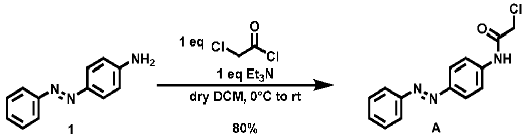

### (E)-2-chloro-N-(4-(phenyldiazenyl)phenyl)acetamide (Switch A)¹

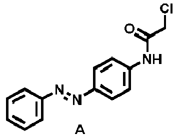

Compound 1 (0.30 mmol, 60 mg) was dissolved in dry dichloromethane (DCM) (8 mL) and the solution cooled to 0 °C. Triethylamine (1.0 eq, 0.30 mmol, 42 µL) was added and the solution was stirred vigorously. Chloroacetyl chloride (1.0 eq, 0.30 mmol, 35 mg) was dissolved in dry DCM (8 mL) and cooled to 0 °C prior to addition and subsequently the reaction mixture was left to stir and warm up to room temperature overnight. The mixture was quenched with water and extracted with DCM (3x 8 mL). The organic layers were washed with 10% aq. HCl and water, dried over MgSO₄ and the solvent was evaporated. The product was purified by flash chromatography on silica gel (hexane : EtOAc = 6:4) to provide 77 mg of product **A** as a orange solid (yield = 80 %). **Mp.** 150-153 °C. ¹**H NMR (600 MHz, DMSO-*d*₆)** δ 10.65 (s, 1H), 7.93 - 7.81 (m, 6H), 7.59 (t, *J* = 7.0 Hz, 2H), 7.55 (t, *J* = 7.9 Hz, 1H), 4.32 (s, 2H). **¹³C NMR (600 MHz, DMSO-*d₆***) δ 165.1, 152.0, 147.9, 141.5, 131.2, 129.4, 123.7, 122.4, 119.6, 43.6. **HRMS** (ESI+) calc for C₁₄H₁₂CIN₃OH: 274.0742, found 274.0752. The spectroscopic data is in accordance with the literature.¹

### Switch B

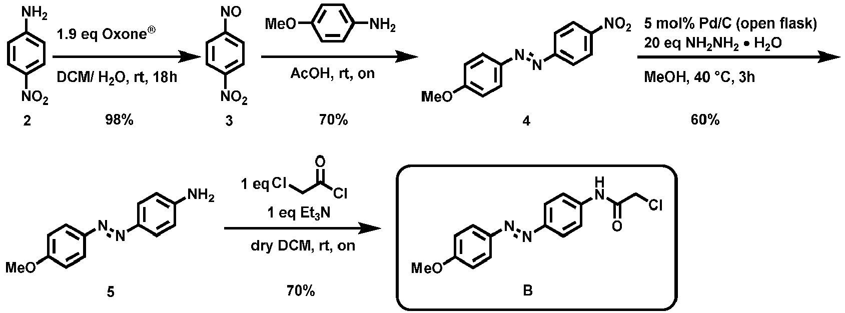

### 1-nitro-4-nitrosobenzene (3)^{2,3}

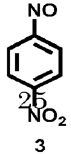

p-Nitroaniline 2 (1.5 mmol, 200 mg) was dissolved in DCM (2 mL). Oxone® (1.95 eq, 5.64 mmol, 1.74 g) was dissolved in water (8 mL) and subsequently added to the aniline solution. The reaction mixture was stirred vigorously overnight at room temperature. The mixture was extracted with DCM, the organic phase was washed with 1M aq. HCl solution, dried over MgSO₄ and the solvent was evaporated. The product was purified by flash column chromatography on silica gel (hexane : EtOAc = 5:1) to provide 240 mg of product **3** as a brown solid (yield = 98%). **Mp.** 126-127 °C. **¹H NMR (600 MHz, CDCl₃**) δ 8.05 (d, *J*=8.79 Hz, 1H), 8.51 (d, *J*=8.75 Hz, 1H). The spectroscopic data is in accordance with the literature. 2,3

### (E)-1-(4-methoxyphenyl)-2-(4-nitrophenyl)diazene (4)^{2,3}

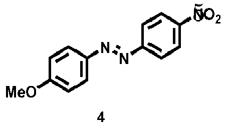

Compound **3** (1.5 mmol, 230 mg) and *p*-methoxyaniline (1.0 eq, 1.5 mmol, 190 mg) were dissolved in glacial acetic acid (14 mL) and the reaction mixture was stirred overnight at room temperature. The crude mixture was extracted with hexane, the organic phase was washed with aqueous NaHCO₃ and dried over MgSO₄. The product was purified by flash column chromatography to yield 380 mg of azobenzene 4 as a brown solid (yield = 70%). **Mp.** 154 °C. **¹H NMR (600 MHz, CDCl₃**) δ 3.93 (s, 3H), 7.05 (d, *J*=8.96 Hz, 2H), 7.99 (m, 4H), 8.37 (d, J= 8.97 Hz, 2H). The spectroscopic data is in accordance with the literature.^{2,3}

### (E)-4-((4-methoxyphenyl)diazenyl)aniline (5)⁴

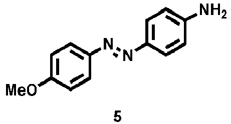

Compound 4 (0.4 mmol, 100 mg) was dissolved in methanol (5 ml) and palladium on carbon (5 mol%, 0.02 mmol, 5 mg, 10 wt%) was added. Hydrazine (20 eq, 8 mmol, 0.4 mL) was added dropwise to the solution. An open condenser was attached to a three-neck flask used as reaction vessel. The heterogeneous orange reaction mixture was left to stir at 40 °C for 3 h and with time became dark brown. The solvent was removed on the rotary evaporator to obtain a green solid. Product **5** was purified by flash column chromatography to provide 60 mg as a orange solid (yield = 60%). **Mp.**140-142 °C. **¹H NMR (400 MHz, CDCl₃**) δ 7.85 (d, *J* = 8.9 Hz, 2H), 7.77 (d, *J* = 8.6 Hz, 2H), 6.99 (d, J = 8.9 Hz, 2H), 6.74 (d, J = 8.6 Hz, 2H), 4.07 (bs, 2H), 3.88 (s, 3H). The spectroscopic data is in accordance with the literature.⁴

### (E)-2-chloro-N-(4-((4-methoxyphenyl)diazenyl)phenyl)acetamide (Switch B)

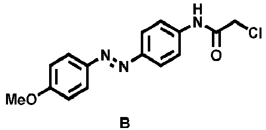

Azobenzene **5** (0.13 mmol, 29 mg) and trimethylamine (1 eq, 0.13 mmol, 17 µL) were dissolved in dry DCM (3.3 mL) and the mixture stirred at 0 °C. Chloroacetyl chloride (1 eq, 0.13 mmol, 10 µL) was dissolved in dry DCM (3.3 mL) and added to the reaction mixture. The pale-yellow reaction mixture changed color to dark orange upon addition. The mixture was stirred for 3.5 h at rt. Subsequently water was added to quench the reaction and the product was extracted with DCM, the organic solution washed with 1 M aq. HCl and water and dried over MgSO₄. The crude product was purified by flash column chromatography on silica gel to yield 30 mg of product **B**as a orange solid (yield = 70%). **Mp.** 166-170 °C. ¹**H NMR (600 MHz, DMSO-*d*₆)** δ 10.61 (s, 1H), 7.89 - 7.85 (m, 4H), 7.80 (d, *J* = 8.9 Hz, 2H), 7.14 (d, *J* = 9.0 Hz, 2H), 4.31 (s, 2H), 3.87 (s, 3H). **¹³C NMR (600MHz, DMSO-*d*₆)** δ 165.0, 161.7, 148.0, 146.2, 140.8, 124.3, 123.3, 119.6, 114.6, 55.6, 43.6. **HRMS** (ESI+) calc for C₁₅H₁₄ClN₃O₂H: 304.0847, found 304.0862.

### Switch C

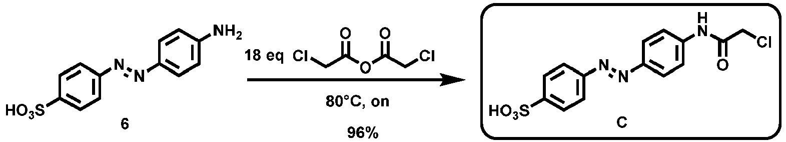

### (E)-4-((4-(2-chloroacetamido)phenyl)diazenyl)benzenesulfonic acid (Switch C)⁵

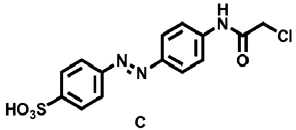

A mixture of azobenzene **6** (0.7 mmol, 200 mg) and chloroacetic anhydride (18 eq, 12 mmol, 2.1 g) was heated up to 90 °C and left to stir overnight in a Schlenk flask. The solution was diluted with DCM and the solid was formed and separated by centrifugation. The crude product was purified by reversed phase column chromatography on C18 silica (water : acetonitrile = 9:1) to provide 240 mg of product **C** as an orange solid (yield = 95%). **Mp.** > 250 °C. **¹H NMR (600 MHz, DMSO-*d*₆)** δ 10.66 (s, 1H), 7.93 (d, *J* = 8.9 Hz, 2H), 7.85 - 7.81 (m, 3H), 7.78 (d, *J* = 8.5 Hz, 2H), 4.32 (s, 2H). **¹³C NMR (600 MHz, DMSO-*d*₆)** δ 165.6, 152.1, 151.2, 148.4, 142.0, 127.2, 124.3, 122.4, 120.1, 44.1. **HRMS** (ESI-) calc for C₁₄H₁₁ClNaO₄S: 352.0153, found 352.0169. The spectroscopic data is in accordance with the literature.⁵

### Switch D

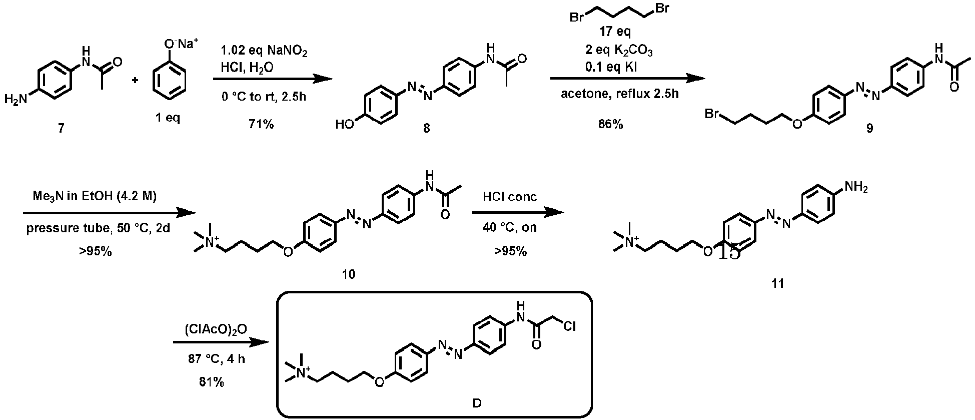

### (E)-N-(4-((4-hydroxyphenyl)diazenyl)phenyl)acetamide (8)⁶

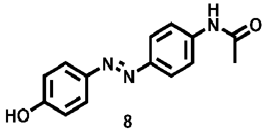

4-Amino-acetaniline (1.00 g, 6.59 mmol, 1.04 eq) was dissolved in ice-cold 6 M HCl (aq) (7 mL) and subsequently added dropwise to a solution of NaNO₂ (470 mg, 6.81 mmol, 1.02 eq) in water (1.5 mL), to which a few crystals of urea were added. The resulting brown mixture was slowly added to ice-cold solution of phenol (610 mg, 6.48 mmol, 1.0 eq) and NaOH (1.26 g, 31.5 mmol, 4.7 eq) in water (4.5 mL). The mixture was stirred for 2.5 h, left to warm up to room temperature and acidified with 6 M HCl to pH=6. The precipitate was filtered off with cold water and dried to provide 1.18 g of compound **8** as a brown solid (yield = 71%). **Mp.** 195 °C. **¹H NMR (400 MHz, DMSO-*d*₆)** δ 10.20 (s, NH), 7.72-7.76 (m, 6H), 6.90 (d, *J* = 8.79 Hz, 2H), 2.07 (s, 3H). The spectroscopic data is in accordance with the literature.⁶

### (E)-N-(4-((4-(4-bromobutoxy)phenyl)diazenyl)phenyl)acetamide (9)⁶

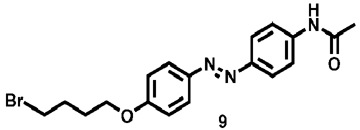

Compound **8** (500 mg, 1.96 mmol), 1,4-dibromobutane (3.92 mL, 32.8 mmol, 17 eq), potassium carbonate (540 mg, 3.91 mmol, 2 eq) and potassium iodide (69.8 mg, 0.420 mmol, 0.1 eq) were dissolved in acetone (20 mL) and the mixture heated under reflux for 3.5 h. After the reaction mixture cooled down, it was diluted with EtOAc (200 mL). The solution was washed with brine (2 x 200 mL), dried over MgSO₄ and the solvent was evaporated. The resulting solid was dissolved in minimum amount of EtOAc to be precipitated by addition of pentane. The solid was filtered and dried to yield 660 mg of compound 9 as a orange solid (yield = 86%). **Mp.** 172-174 °C. **¹H NMR (400 MHz, DMSO-*d*₆)** δ 10.24 (s, NH), 7.76-7.85 (m, 6H), 7.11 (d, *J* = 8.84 Hz, 2H), 4.12 (t, *J*= 6.2 Hz, 2H), 3.63 (t, *J*=5.89 Hz. 2H), 2.09 (s, 3H), 2.03-1.96 (m, 2H), 1.93-1.84 (m, 2H). The spectroscopic data is in accordance with the literature.⁶

### (E)-4-(4-((4-acetamidophenyl)diazenyl)phenoxy)-N,N,N-trimethylbutan-1-aminium chloride (10)

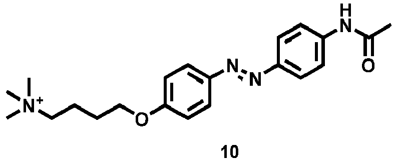

Azobenzene **9** (250 mg, 0.641 mmol) was dissolved in trimethylamine (4.2 M in EtOH, 15 mL) in a pressure tube and left to stir for 2 d at 50 °C. The vial was cooled down and diethyl ether was added (250 mL). The precipitate was filtered off and washed several times with diethyl ether to provide 230 mg compound **10** as a yellow solid (yield = 97%). **Mp.** >250 °C. **¹H NMR (400 MHz, DMSO-*d*₆)** δ 10.27 (s, NH), 7.87-7.76 (m, 6H), 7.13 (d, *J* = 8.92 Hz, 2H), 4.14 (t, *J* = 5.94 Hz, 2H), 3.41-3.36 (m, 2H), 3.07 (s, 9H), 2.10 (s, 3H), 1.93-1.84 (m, 2H), 1.83-1.74 (m, 2H). **¹³C NMR (600 MHz, DMSO-*d*₆)** δ 168.7, 160.8, 147.4, 146.3, 141.8, 124.2, 123.2, 119.1, 115.0, 67.2, 65.0, 52.2, 25.5, 24.1, 19.2. **HRMS** (ESI+) calc for C₁₄H₁₁ClNaO₄: 369.2285, found 369.2288.

### (E)-4-(4-((4-aminophenyl)diazenyl)phenoxy)-N,N,N-trimethylbutan-1-aminium chloride (11)

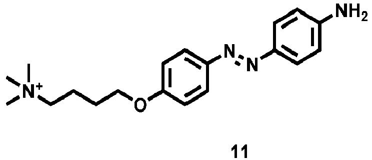

Compound **10** (200 mg, 0.541 mmol) was dissolved in conc. HCl (15 mL) and left to stir at 40 °C overnight. HCl was evaporated with multiple additions of water and finally the reaction mixture, diluted with water, was freeze-dried. Compound **11** (180 mg) was obtained as a pale yellow solid (quant. yield). **Mp.** >250 °C. **¹H NMR (600 MHz, DMSO-*d*₆)** δ 7.79 (d, *J* = 8.86 Hz, 2H), 7.71 (d, *J* = 8.64 Hz, 2H), 7.10 (d, *J* = 8.93 Hz, 2H), 6.93 (d, *J* = 8.47 Hz, 2H), 4.12 (t, *J* = 5.93, 2H), 3.42-3.37 (m, 2H), 3.08 (s, 9H), 1.91-1.84 (m, 2H), 1.81-1.74 (m, 2H). **¹³C NMR (600 MHz, DMSO-*d*₆)** δ 160.2, 148.7, 146.5, 146.3, 143.2, 126.1, 124.4, 123.8, 123.6, 114.9, 114.7, 67.09, 65.0, 52.2, 25.6, 19.2. **HRMS** (ESI+) calc for C₁₉H₂₇N₄O: 327.2179, found 327.2181. Compound **11** was used without further purification in the next step.

### (E)-4-(4-((4-(2-chloroacetamido)phenyl)diazenyl)phenoxy)-N,N,N-trimethylbutan-1-aminium chloride (Switch D)

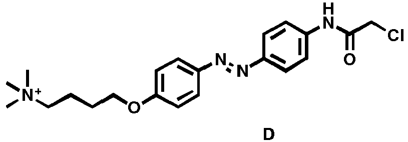

Compound **11** (150 mg, 0.46 mmol) was mixed with chloroacetic anhydride (450 mg, 2.63 mmol, 5.7 eq) in a Schlenck tube and heated to 87 °C under N₂ for 4 h. After cooling the obtained sticky brown solid was washed five times with cold ether by using centrifugation to separate the solid from excess chloroacetic anhydride. The obtained product was dissolved in water and freeze-dried to acquire 150 mg of compound **D** as a pale yellow solid (yield = 81%). **Mp.** >250 °C. **¹H NMR (600 MHz, DMSO-*d*₆)** δ 10.73 (s, 1H), 7.78-7.85 (m, 4H), 7.82 (d, *J* = 8.93Hz, 2H), 7.15 (d, *J* =9 Hz, 2H), 4.33 (s, 2H), 4.19-4.14 (m, 2H), 3.41-3.38 (m, 2H), 3.08 (s, 9H), 1.92-1.86 (m, 2H), 1.78-1.86 (m, 2H). **¹³C NMR (600 MHz, DMSO-*d*₆)** δ 168.7. 162.1, 152.6, 146.7, 138.8, 124.8, 123.8, 120.1, 115.7, 67.8, 65.4, 52.8, 44.1, 26.0, 19.7. **HRMS** (ESI+) calc for C₂₁H₂₈ClN₄O₂: 403.1895, found 403.1895.
A.

### Cloning of cysteine mutants of FraC

S-FraC-Y138C was prepared by using 100 ng pT7-SC1 plasmid containing the WtFraC gene with the additional mutation W112S (S-FraC) that enhances expression in *E. coli* cells⁸ and a His₆-tag at the C-terminus for purificationas template for the PCR reaction. The gene was amplified by using REDTaq ReadyMix, 2 µM of Y138Cr and T7 promotor primers (Table 1) in 200 µL reaction volume. The PCR reaction cycling protocol was as following: a pre-incubation step at 98 °C for 90 sec was followed by 30 cycles of denaturation at 98 °C for 15 sec, annealing at 55 °C for 15 sec and extension at 72°C for 2 min. The PCR product was purified using QIAQuick PCR purification kit (Qiagen) and cloned into pT7-SC1 by the MEGAWHOP procedure⁹ using 300 ng pT7-SC1 plasmid containing S-FraC gene as template and 500 ng of the purified PCR product as primers. The amplification was carried out with Phire Hot Start II DNA polymerase (Finnzymes) in 50 µL final volume (pre-incubation at 98 °C for 30 sec, then 30 cycles of: denaturation at 98°C for 5 sec, extension at 72 °C for 1.5 min). The circular template was eliminated by incubation with Dpn I (1 FDU) for 2 h at 37 °C. Of the resulting mixture 0.6 µL was transformed into E. cloni® 10G cells (Lucigen) by electroporation. The transformed bacteria were grown overnight at 37 °C on ampicillin (100 µg/ml) containing lysogeny broth (LB) agar plates. The identity of the clones were confirmed by sequencing.

The other cysteine mutants of FraC were prepared as described above using the corresponding reverse primers (G13Cr, K77Cr, S112Cr, Q130Cr, E134Cr, G145Cr, N147Cr and S166Cr, Table 1) for the first PCR reaction.

**Table 1: primers used**

| **Name of primer** | **DNA sequence (5'→3')** |
|---|---|
| G13Cr | CAAAGCCCAGGCACGCACCGTCG |
| K77Cr | GACCGCGATTGCATTGACCG |
| W112Cr | CCAATTGCTATAGCAATTATAATCGTACGGCACG |
| Q130Cr | CTCGTACATGCGGCAATCGGCACG |
| E134Cr | CAGCTCGCAGTACATGCGCTGATCGG |
| Y138Cr | GCGATGGCAGTACAGCTCCTCGTAC |
| G145Cr | GAATGCCAACCGTTGTCGCAGCGAAACGGCGAGC |
| N147Cr | CCAACCGCAGTCGCCGCGAAACGG |
| S166Cr | GATTGCGTGGCCCGAGCAATTCATAAAGCCGCGAC |
| T7-terminator | GCTAGTTATTGCTCAGCGG |
| T7-promoter | TAA T ACGACTCACT A T AGGG |

### >S-FraC (amino acid sequence)

MASADVAGAVIDGAGLGFDVLKTVLEALGNVKRKIAVGIDNESGKTWTA
MNTYFRSGTSDIVLPHKVAHGKALLYNGQKNRGPVATGVVGVIAYSMS
DGNTLAVLFSVPYDYNSYSNWWNVRVYKGQKRADQRMYEELYYHRSP
FRGDNGWHSRGLGYGLKSRGFMNSSGHAILEIHVTKAGSAHHHHHH*

### >S-FraC (nucleotide sequence)

ATGGCGAGCGCCGATGTCGCGGGTGCGGTAATCGACGGTGCGGGTCTG
GGCTTTGACGTACTGAAAACCGTGCTGGAGGCCCTGGGCAACGTTAAA
CGCAAAATTGCGGTAGGGATTGATAACGAATCGGGCAAGACCTGGACA
GCGATGAATACCTATTTCCGTTCTGGTACGAGTGATATTGTGCTCCCAC
ATAAGGTGGCGCATGGTAAGGCGCTGCTGTATAACGGTCAAAAAATC
GCGGTCCTGTCGCGACCGGCGTAGTGGGTGTGATTGCCTATAGTATGT
CTGATGGGAACACACTGGCGGTACTGTTCTCCGTGCCGTACGATTATAA
TAGCTATAGCAATTGGTGGAACGTGCGTGTCTACAAAGGCCAGAAGCG
TGCCGATCAGCGCATGTACGAGGAGCTGTACTATCATCGCTCGCCGTTT
CGCGGCGACAACGGTTGGCATTCCCGGGGCTTAGGTTATGGACTCAAA
AGTCGCGGCTTTATGAATAGTTCGGGCCACGCAATCCTGGAGATTCAC
GTTACCAAAGCAGGCTCTGCGCATCATCACCACCATCACTGA

### FraC Protein expression, photoswitch modification and purification

*E. cloni*® EXPRESS BL21 (DE3) cells were transformed with the pT7-SC1 plasmid containing the appropriate FraC cysteine mutant gene of Example 3. Transformants were selected after overnight growth at 37 °C on LB agar plates supplemented with 100 mg/L ampicillin. The resulting colonies were inoculated in 2x YT medium containing 100 mg/L of ampicillin. The culture was grown at 37 °C, with shaking at 200 rpm, until it reached an OD₆₀₀ of ∼0.6. Protein expression of protein was induced by the addition of 0.5 mM isopropyl-β-D-thiogalactopyranoside and the growth was continued at 20 °C. The next day the bacteria were harvested by centrifugation at 6000 xg for 30 min and pellets were stored at -80 °C.

The pellets containing overexpressed mutant FraC proteins were thawed and resuspended in 20 mL of 15 mM Tris.HCl pH 7.5, 150 mM NaCl, 4 M urea, 20 mM imidazole, 1 mM MgCl₂ and 0.05 units/mL of DNase I (Fermentas). Additionally, to avoid oxidation of the cysteine residues, 5 mM Tris(2-carboxyethyl)phosphin (TCEP) was added to the solution.

The bacteria suspension was then supplemented with 0.2 mg/mL lysozyme to initiate cell disruption, followed by vigorous shaking at ambient temperature for 1 h. The remaining bacteria were disrupted by probe sonication. The crude lysates were clarified by centrifugation at 6000 xg for 30 min and supernatant mixed with 200 µL (bead volume) of Ni-NTA resin (Qiagen) that was pre-equilibrated with wash buffer (20 mM imidazole, 150 mM NaCl, 15 mM Tris.HCl pH 7.5). After 45 min of gentle mixing at ambient temperature, the resin was loaded onto a column (Micro Bio Spin, Bio-Rad) and washed first with ∼10 mL of wash buffer supplemented with 5 mM TCEP and then washed with ∼10 mL of wash buffer without reducing agent.

Immediately after washing, FraC monomers bound to the Ni-NTA resin were mixed with ∼100-fold molar excess of azobenzene photoswitch, dissolved, 15 mM Tris.HCl pH 7.5,150 mM NaCl in dimethyl sulfoxide (DMSO, final 20%) and the reaction was allowed to proceed at room temperature overnight in the dark. The following day the resin was washed again with ∼10 mL of wash buffer supplemented with 5 mM TCEP to reduce unmodified cysteines and then washed with ∼10 mL of wash buffer without reducing agent to remove free azobenzene molecules.

Then, modified FraC monomers were eluted with approximately 150 µl of 200 mM ethylenediaminetetraacetic acid (EDTA), 15 mM Tris.HCl pH 7.5 and 150 mM NaCl. To assess the extent of photoswitch modification, an aliquot of FraC-azobenzene and the unmodified FraC monomers were treated with 10x PEG-5000-maleimide. The reaction mixture was incubated for 3 h at room temperature and then analyzed by SDS-PAGE electrophoresis.

FraC monomers conjugated to the photoswitch were separated from unmodified monomers by cation exchange chromatography. Modified monomer solutions were first dialyzed for 3 h against buffer A (50 mM Tris.HCl pH 9.5, 2 M urea and 5 mM β-mercaptoethanol, total volume 1500 mL, buffer replaced two times) to reduce the salt concentration and bring the pH to 9.5. The sample was then applied to a HiTrap SP HP column (GE Healthcare Life Science) connected to Äkta pure chromatography system (GE Healthcare Life Science) equilibrated with buffer A. The protein was eluted with a gradient of buffer B (1 M NaCl and 50 mM Tris.HCl pH 9.5), starting with a step to 5 % and continuing with raising B from 5 to 40 % in 80 min, and then a final step to 100 %.

Protein elution was monitored by measuring absorbance at 280 nm and 365 nm. While the first peak at 19.5 % B corresponds to FraC-Y138C-C (280 nm and 365 nm absorbance) monomer, the second peak correspond to unmodified FraC monomers (only 280 nm absorbance). Collected fractions of the first peak were combined and concentrated using Amicon Ultra Centrifugal Filters (10 kDa cut-off). Protein concentration was determined by Bradford assay. All monomers were stored at 4 °C until further use.

### Hemolytic activity assay

Defibrinated sheep blood (ThermoFisher Scientific) was washed with 150 mM NaCl, 15 mM Tris.HCl pH 7.5 until the supernatant was clear. The erythrocytes were then resuspended with the same buffer to ∼1 % concentration (OD₆₅₀ 0.6 - 1.0). The suspension (120 µL) was then mixed with the solutions containing 0.3 nM - 11 µM FraC monomer in *cis* or *trans* state. Photoisomerization of the azobenzene to the cis-state was achieved by illumination for 5 min with a UV lamp (Thorlabs model M365F1 LED, 365 nm, 4.1 mW). Isomerization back to *trans*-state was achieved by irradiation for 2 minutes with white light (Thorlabs OSL1-EC Fiber Illuminator, >450nm, 150 W halogen lamp, output 40000 foot-candles).

Hemolytic activity was measured by monitoring the decrease in OD₆₅₀ using a MultiskanTM GO Microplate spectrophotometer (ThermoFisher Scientific) or Synergy H1 Hybrid-Multimode reader (BioTek). Percentage of hemolysis was calculated as followed % hemolysis = 100^{∗}(Abs_{C-FraC}-Abs_{buffer})/(Absₜᵣᵢₜₒₙ-Abs_{buffer}). Experiments were performed in the dark.

### Cell viability assay

The cell viability assays were performed with the Human squamous carcinoma cell line A431 (Sigma-Aldrich). Detached cell number was determined by cell counting using an improved Neubauer chamber. The viability of cells was measured using (2-2-methoxy-4-nitrophenyl)-5-(2,4-disulfophenyl)-2H-tetrazolium, monosodium salt (WST-8) based cell viability assay included in the Cell Counting Kit-8 (CCK,-8, Sigma-Aldrich) according to the manufactures instructions.^{10,11}

The human squamous carcinoma cell line A431 (Sigma-Aldrich) was routinely maintained at 37 °C, in a humidified incubator under 5% CO₂. A431 cells were grown in Dulbecco's Modified Eagle Medium (DMEM) supplemented with 10 % Fetal Bovine Serum (FBS), 2 mM glutamine, 100 µg mL⁻¹ gentamycin.

A431 cells were first detached with 5 mL of 0.25 % Trypsin/EDTA solution, centrifuged at 400 xg for 5 minutes, and suspended in 10 mL growth medium, counted as described above and seeded 20.000 cells per well in 96-well tissue culture plates in 200 µL growth medium and incubated overnight at 37 °C in 5 % CO₂ atmosphere.

10 µL of FraC monomer solution in various concentrations (1.2 nM to 3.6 µM) was distributed to the cells and incubated 2 hours at 37 °C in a 5 % CO₂ atmosphere. Cells with only medium were included as control. Next, 5 µL CCK-8 solution was added to each well and the plates were incubated 2 hours before measuring absorbance at 450 nm using the Synergy H1 Hybrid-Multimode reader (BioTek). Experiments were performed in the dark. Results are shown as percentage of control cells and represented as average ± standard deviation of 3 independent experiments with triplicates.

### EXAMPLE 1: Design of photo-controlled FraC nanopore

In order to design a photo-controlled FraC nanopore, we focused on the interaction of the protein with the lipid cell membrane. The aim was to obtain a toxin for which lipid binding was inhibited when the protein-appended photoswitch was in the resting *trans* state, while promoting lipid binding and subsequent pore formation upon photoswitching into the *cis* isomer.

Nine cysteine substitutions were tested (Figure 2D): three residues (Q130, E134 and Y138), which are part of the a2 helix of FraC, were selected because their side chains pointed directly towards the membrane. K77 ⁵³ and W112^{49,54,55} were chosen since their substitution was reported to modulate hemolytic activity and membrane binding. G145, N147 and S166 were selected for their location in a loop at the protomer interface that faces the intercalated sphingomyelin lipid. Many of such residues were also close or in direct contact with sphingomyelin (Figure 2D), a lipid molecule that triggers FraC oligomerization (Figure 1A).^{55,56} Finally, G13 was chosen because it is in the transmembrane spanning helix (α1) and points towards the neighboring protomer.

For the protein modification, four azobenzene switches were designed for covalent attachment to a cysteine-modified FraC *via* a reactive chloroacetyl moiety (Figure 2A). The switches were designed with different substituents at the *para* position, where the first compound had no additional substituents (switch A), the second a methoxy group in order to improve the photoswitching properties⁵⁷ (switch B), the third a water-soluble negatively charged sulfonate group (switch C) and the last a positively charged quaternary ammonium group (switch D).

The switches were synthesized as described herein above based on previously described protocols and their switching properties were studied by NMR and UV-Vis spectroscopy (Figure 2B and C). Irradiation at λ = 365 nm switched the azobenzene moiety from *trans* to the *cis* state, as observed by the decrease in the absorbance at 355 nm and the emergence of new signals in the ¹H NMR spectrum (Figure 2C, peak F). The photostationary state ratio (PSS ratio), after achieving dynamic equilibrium under irradiation with 365 nm light, was then calculated by relative integration of the methylene peaks (Figure 2C, peak F). All four molecules proved to have high PSS ratios, over 90:10, when switching to the *cis* isomer . However, since switches A and B were poorly soluble in the attachment buffer used, azobenzene switches C and D were selected for further studies.

Azobenzene switch C was covalently attached to all different cysteine residues introduced in FraC (as confirmed by Mass Spectrometry, Figure 2D). The modified nanopores showed an additional absorbance peak at λ = 365 nm, typical for the azobenzene moiety (Figure 2E), and the reversible *trans* to *cis* isomerization could be followed upon irradiation at λ = 365 nm and white light (>450 nm). Numerous switching cycles could be performed, indicating the stability of the construct under aqueous conditions (Figure 2G). The cytotoxicity of the different constructs was tested on red blood cells (hemolytic activity) before and after activation by irradiation with λ = 365 nm light (Figure 2H). Modification at positions 145 and 147 (FraC-G145C-C and FraC-N147C- C, respectively) resulted in inactive molecules (i.e. they showed no hemolytic activity). Since residues G145 and N146 are both at the interface between protomers, it is conceivable that the azobenzene moiety prevented the oligomerization of FraC.

For other tested positions, the attachment of the photoswitch to the substituted cysteine residue resulted in a reduction in the activity of the protein, with notable exception being the attachments at positions 130 and 134 (FraC-Q130C- C and FraC-E134C-C, respectively, see Figure 3). Both residues are in the a2 helix, which resides right above the membrane (Figure 2D), suggesting that hydrophobic interactions of the a2 helix facilitate the docking of FraC with the lipid bilayer. Among the variants that were hemolytically active, modifications at position 13 and 130 showed only a small difference between the *cis* and *trans* isoforms. Modification at position 77 and 166 showed a higher cytotoxicity for the *trans* than for the *cis* isoform, while modifications at position 134, 112 and 138 showed a faster hemolytic activity for the *cis* compared to the *trans* isoform (Figure 2H and Figure 3).

### EXAMPLE 2: Characterization of an exemplary FraC nanopore

Given the interest, e.g. for use as a cytotoxic drug, to obtain a toxin that is activated upon light irradiation, we selected FraC-Y138C-C , FraC-W112C-C and FraC-E134C-C for further characterization. The photoswitch-modified monomers were separated from unmodified FraC monomers using cation exchange chromatography (Figure 4). Thereafter, the HC₅₀, which is the hemolytic concentration of toxin necessary to obtain 50% red blood cell lysis, was tested for the *cis* and *trans* isoforms. Although all constructs were more active in the *cis* configuration (data not shown) FraC-Y138C-C showed the largest difference between the *cis* and *trans* HC₅₀ [1.65 ± 0.10 µM and 6.38 ± 0.69 µM, respectively] (Figure 5). Notably, 2.2 µM of FraC-Y138C-C showed no hemolytic activity, while irradiation with 365 nm light prior to addition induced complete red blood cell lysis (Figure 5A), indicating a successful design of a photocontrolled FraC nanopore.

Furthermore, the activated pore could be switched off again through irradiation with white light, (Figure 5B). This reversibility was further expanded to the assay conditions, since active toxins could be deactivated in the presence of red blood cells by irradiation with white light *in situ* (Figure 5C), indicating that the cytotoxicity is reversibly controlled by light. Notably, substitution of the negatively charged sulfonate group with a positively charged quaternary ammonium group also showed *cis* versus *trans* selectivity (Figure 6).

### EXAMPLE 3: Photo-controlled cellular toxicity

The cytotoxicity of FraC-Y138C-C towards human cancer cells was investigated to test its usefulness as a chemotherapeutic agent. The addition of up to 1.1 µM *trans* FraC-Y138C-C to A431 epidermoid carcinoma cells did not cause any cell death, while an equal concentration of *cis* showed cytotoxicity [HC₅₀ = 0.93 ± 0.05 µM] (Figure 5D). This result indicates that light activated toxins can function towards different types of eukaryotic cells. Furthermore, at the concentration in which the *cis* isomer showed complete killing of A431 cancer cells, the *trans* isomer did not show any hemolytic activity towards healthy red blood cells, pointing towards the usefulness of the construct in photoactivated tumor therapy.

### EXAMPLE 4: Analytical application of photo-controlled FraC nanopore

Alongside potential use as a cancer therapeutic, a photo-controlled FraC nanopore of the invention is advantageously used as a single-molecule sensor. In its oligomeric form, FraC forms nanoscale pores (nanopores) in artificial lipid bilayers. Under an applied transmembrane potential, the induced ionic current across single nanopores (Figure 7A) was used to identify and study single molecules, including DNA⁴⁸, peptides and proteins^{49,50}. Most notably, arrays of thousands of nanopores are now embedded into commercial DNA sequencing devices. Insertion of a single nanopore in a sensing device, however, is a stochastic process that cannot be fully controlled. Thus, a nanopore whose bilayer insertion can be controlled with light would speed up analysis and reduce the cost of multiplexing in nanopore sensing devices.

Therefore, we tested the ability of our photoswitch-modified FraC monomers to reversibly form nanopores in lipid bilayers. It was found that the *trans* isomer of FraC-Y138C-C could not form nanopores (Figure 7B), while direct irradiation of the electrophysiology chamber with 365 nm light, forming the *cis* isomer, activated the nanopores as shown by stepwise increases of the bilayer conductance reflecting individual insertions into the planar lipid bilayer (Figure 7C). Subsequent *in situ* irradiation with white light almost completely inhibited further assembly of FraC nanopores (Figure 7D).

### EXAMPLE 5: Synthesis of a visible light responsive photoswitch

Modification of the azobenzene core by introducing four fluorine atoms in the *ortho* positions enables a red shift in the absorption spectrum of the molecule allowing reversible switching of the molecule with blue (500 nm) and green (410 nm) light irradiation. Therefore we aimed to synthesize photoswitch **C** as a visible light controlled azobenzene by introducing four fluorine moieties in the *ortho* positions. Molecule **1,** containing both the fluoro and bromo substituents in the *para* positions for functionalization, was prepared via the modified Mills reaction. The water-solubilizing group, sulfonate, was installed via an aromatic nucleophilic substitution reaction with the fluorine atom in the *para* position. The sulfonated product underwent a Buchwald-Hartwig coupling with acetimide to install the amide which was subsequently hydrolysed before introducing the chloroacetyl moiety resulting in the water-soluble visible light responsive switch **5.** 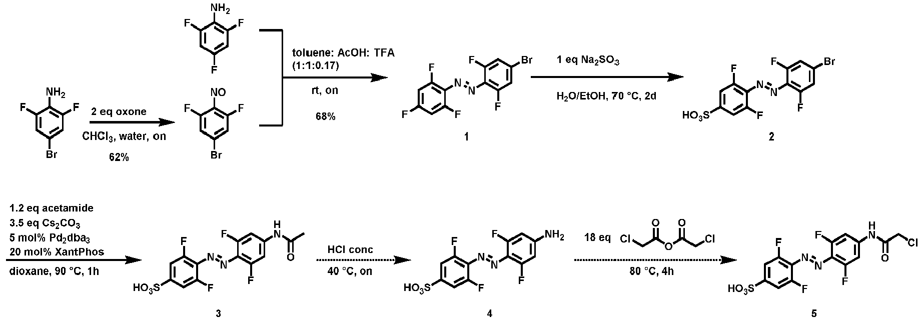 Synthetic scheme of a visible light controlled photoswitch

Switch **5** can be attached to a FraC monomer, e.g. via a free cysteine moiety, yielding a modified FraC monomer capable of forming a FraC nanopore that can be reversibly controlled by visible light.

### REFERENCES

(1) Velema, W. A.; Szymanski, W.; Feringa, B. L. Photopharmacology: Beyond Proof of Principle. J. Am. Chem. Soc. 2014, 136 (6), 2178-2191.
(2) Hüll, K.; Morstein, J.; Trauner, D. In Vivo Photopharmacology. Chem. Rev. 2018, 118 (21), 10710-10747.
(3) Lerch, M. M.; Hansen, M. J.; van Dam, G. M.; Szymanski, W.; Feringa, B. L. Emerging Targets in Photopharmacology. Angew. Chemie Int. Ed. 2016, 55 (37), 10978-10999.
(4) Pianowski, Z. L. Recent Implementations of Molecular Photoswitches into Smart Materials and Biological Systems. Chemistry 2019, 25 (20), 5128-5144.
(5) Wang, J.; Hou, L.; Browne, W. R.; Feringa, B. L. Photoswitchable Intramolecular Through-Space Magnetic Interaction. J. Am. Chem. Soc. 2011, 133 (21), 8162-8164.
(6) Szymański, W.; Beierle, J. M.; Kistemaker, H. A. V.; Velema, W. A.; Feringa, B. L. Reversible Photocontrol of Biological Systems by the Incorporation of Molecular Photoswitches. Chem. Rev. 2013, 113 (8), 6114-6178.
(7) Tochitsky, I.; Kienzler, M. A.; Isacoff, E.; Kramer, R. H. Restoring Vision to the Blind with Chemical Photoswitches. Chem. Rev. 2018, 118 (21), 10748-10773.
(8) Beharry, A. A.; Woolley, G. A. Azobenzene Photoswitches for Biomolecules. Chem. Soc. Rev. 2011, 40 (8), 4422-4437.
(9) Hüll, K.; Morstein, J.; Trauner, D. In Vivo Photopharmacology. Chem. Rev. 2018, 118 (21), 10710-10747.
(10) Koçer, A.; Walko, M.; Bulten, E.; Halza, E.; Feringa, B. L.; Meijberg, W. Rationally Designed Chemical Modulators Convert a Bacterial Channel Protein into a PH-Sensory Valve. Angew. Chemie Int. Ed. 2006, 45 (19), 3126-3130.
(11) Fortin, D. L.; Banghart, M. R.; Dunn, T. W.; Borges, K.; Wagenaar, D. A.; Gaudry, Q.; Karakossian, M. H.; Otis, T. S.; Kristan, W. B.; Trauner, D.; et al. Photochemical Control of Endogenous Ion Channels and Cellular Excitability. Nat. Methods 2008, 5 (4), 331-338.
(12) Levitz, J.; Broichhagen, J.; Leippe, P.; Konrad, D.; Trauner, D.; Isacoff, E. Y. Dual Optical Control and Mechanistic Insights into Photoswitchable Group II and III Metabotropic Glutamate Receptors. Proc. Natl. Acad. Sci. U. S. A. 2017, 114 (17), E3546-E3554.
(13) Zhang, F.; Sadovski, O.; Xin, S. J.; Woolley, G. A. Stabilization of Folded Peptide and Protein Structures via Distance Matching with a Long, Rigid CrossLinker. J. Am. Chem. Soc. 2007, 129 (46), 14154-14155.
(14) Samanta, S.; Qin, C.; Lough, A. J.; Woolley, G. A. Bidirectional Photocontrol of Peptide Conformation with a Bridged Azobenzene Derivative Supporting Information. Angew. Chemie - Int. Ed.
(15) Ferreira, R.; Nilsson, J. R.; Solano, C.; Andréasson, J.; Grøtli, M. Design, Synthesis and Inhibitory Activity of Photoswitchable RET Kinase Inhibitors. Sci. Rep. 2015, 5 (1), 9769.
(16) Volgraf, M.; Gorostiza, P.; Szobota, S.; Helix, M. R.; Isacoff, E. Y.; Trauner, D. Reversibly Caged Glutamate: A Photochromic Agonist of Ionotropic Glutamate Receptors. J. Am. Chem. Soc. 2007, 129 (2), 260-261.
(17) Matera, C.; Gomila, A. M. J.; Camarero, N.; Libergoli, M.; Soler, C.; Gorostiza, P. Photoswitchable Antimetabolite for Targeted Photoactivated Chemotherapy. J. Am. Chem. Soc. 2018, 140 (46), 15764-15773.
(18) Izquierdo-Serra, M.; Bautista-Barrufet, A.; Trapero, A.; Garrido-Charles, A.; Díaz-Tahoces, A.; Camarero, N.; Pittolo, S.; Valbuena, S.; Pérez-Jiménez, A.; Gay, M.; et al. Optical Control of Endogenous Receptors and Cellular Excitability Using Targeted Covalent Photoswitches. Nat. Commun. 2016, 7 (1), 12221.
(19) Pittolo, S.; Gómez-Santacana, X.; Eckelt, K.; Rovira, X.; Dalton, J.; Goudet, C.; Pin, J.-P.; Llobet, A.; Giraldo, J.; Llebaria, A.; et al. An Allosteric Modulator to Control Endogenous G Protein-Coupled Receptors with Light. Nat. Chem. Biol. 2014, 10 (10), 813-815.
(20) Clapham, D. E. TRP Channels as Cellular Sensors. Nature 2003, 426 (6966), 517-524.
(21) Matsuzaki, T.; Tajika, Y.; Tserentsoodol, N.; Suzuki, T.; Aoki, T.; Hagiwara, H.; Takata, K. Aquaporins: A Water Channel Family. Anat. Sci. Int. 2002, 77 (2), 85-93.
(22) Dal Peraro, M.; van der Goot, F. G. Pore-Forming Toxins: Ancient, but Never Really out of Fashion. Nat. Rev. Microbiol. 2016, 14 (2), 77-92.
(23) Zaydman, M. A.; Silva, J. R.; Cui, J. Ion Channel Associated Diseases: Overview of Molecular Mechanisms. Chem. Rev. 2012, 112 (12), 6319-6333.
(24) Kasianowicz, J. J. Introduction to Ion Channels and Disease. Chem. Rev. 2012, 112 (12), 6215-6217.
(25) Gorostiza, P.; Isacoff, E. Y. Nanoengineering Ion Channels for Optical Control. Physiology (Bethesda). 2008, 23 (5), 238-247.
(26) Bregestovski, P. D.; Maleeva, G. V. Photopharmacology: A Brief Review Using the Control of Potassium Channels as an Example. Neurosci. Behav. Physiol. 2019, 49 (2), 184-191.
(27) Szymański, W.; Yilmaz, D.; Koçer, A.; Feringa, B. L. Bright Ion Channels and Lipid Bilayers. Acc. Chem. Res. 2013, 46 (12), 2910-2923.
(28) Chang, C.; Niblack, B.; Walker, B.; Bayley, H. A Photogenerated Pore-Forming Protein. Chem. Biol. 1995, 2 (6), 391-400.
(29) Koçer, A.; Walko, M.; Meijberg, W.; Feringa, B. L. Chemistry: A Light-Actuated Nanovalve Derived from a Channel Protein. Science (80-.). 2005, 309 (5735), 755-758.
(30) Koçer, A.; Walko, M.; Feringa, B. L. Synthesis and Utilization of Reversible and Irreversible Light-Activated Nanovalves Derived from the Channel Protein MscL. Nat. Protoc. 2007, 2 (6), 1426-1437.
(31) Mourot, A.; Tochitsky, I.; Kramer, R. H. Light at the End of the Channel: Optical Manipulation of Intrinsic Neuronal Excitability with Chemical Photoswitches. Front. Mol. Neurosci. 2013, 6, 5.
(32) Banghart, M. R.; Mourot, A.; Fortin, D. L.; Yao, J. Z.; Kramer, R. H.; Trauner, D. Photochromic Blockers of Voltage-Gated Potassium Channels. Angew. Chem. Int. Ed. Engl. 2009, 48 (48), 9097-9101.
(33) Fehrentz, T.; Huber, F. M. E.; Hartrampf, N.; Bruegmann, T.; Frank, J. A.; Fine, N. H. F.; Malan, D.; Danzl, J. G.; Tikhonov, D. B.; Sumser, M.; et al. Optical Control of L-Type Ca2+ Channels Using a Diltiazem Photoswitch. Nat. Chem. Biol. 2018, 14 (8), 764-767.
(34) Volgraf, M.; Gorostiza, P.; Numano, R.; Kramer, R. H.; Isacoff, E. Y.; Trauner, D. Allosteric Control of an Ionotropic Glutamate Receptor with an Optical Switch. Nat. Chem. Biol. 2006, 2 (1), 47-52.
(35) Rennhack, A.; Grahn, E.; Kaupp, U. B.; Berger, T. K. Photocontrol of the Hv1 Proton Channel. ACS Chem. Biol. 2017, 12 (12), 2952-2957.
(36) Leippe, P.; Winter, N.; Sumser, M. P.; Trauner, D. Optical Control of a Delayed Rectifier and a Two-Pore Potassium Channel with a Photoswitchable Bupivacaine. ACS Chem. Neurosci. 2018, 9 (12), 2886-2891.
(37) Barber, D. M.; Schönberger, M.; Burgstaller, J.; Levitz, J.; Weaver, C. D.; Isacoff, E. Y.; Baier, H.; Trauner, D. Optical Control of Neuronal Activity Using a Light-Operated GIRK Channel Opener (LOGO). Chem. Sci. 2016, 7 (3), 2347-2352.
(38) Bonardi, F.; London, G.; Nouwen, N.; Feringa, B. L.; Driessen, A. J. M. Light-Induced Control of Protein Translocation by the SecYEG Complex. Angew. Chemie Int. Ed. 2010, 49 (40), 7234-7238.
(39) Banghart, M.; Borges, K.; Isacoff, E.; Trauner, D.; Kramer, R. H. Light-Activated Ion Channels for Remote Control of Neuronal Firing. Nat. Neurosci. 2004, 7 (12), 1381-1386.
(40) Fortin, D. L.; Dunn, T. W.; Fedorchak, A.; Allen, D.; Montpetit, R.; Banghart, M. R.; Trauner, D.; Adelman, J. P.; Kramer, R. H. Optogenetic Photochemical Control of Designer K+ Channels in Mammalian Neurons. J. Neurophysiol. 2011, 106 (1), 488-496.
(41) Rullo, A.; Reiner, A.; Reiter, A.; Trauner, D.; Isacoff, E. Y.; Woolley, G. A. Long Wavelength Optical Control of Glutamate Receptor Ion Channels Using a Tetra- Ortho -Substituted Azobenzene Derivative. Chem. Commun. 2014, 50 (93), 14613-14615.
(42) Lemoine, D.; Habermacher, C.; Martz, A.; Mery, P.-F.; Bouquier, N.; Diverchy, F.; Taly, A.; Rassendren, F.; Specht, A.; Grutter, T. Optical Control of an Ion Channel Gate. Proc. Natl. Acad. Sci. 2013, 110 (51), 20813-20818.
(43) Kahlstatt, J.; Reiß, P.; Halbritter, T.; Essen, L. O.; Koert, U.; Heckel, A. A Light-Triggered Transmembrane Porin. Chem. Commun. 2018, 54 (69), 9623-9626.
(44) Mutter, N. L.; Soskine, M.; Huang, G.; Albuquerque, I. S.; Bernardes, G. J. L.; Maglia, G. Modular Pore-Forming Immunotoxins with Caged Cytotoxicity Tailored by Directed Evolution. ACS Chem. Biol. 2018, 13 (11), 3153-3160.
(45) Majd, S.; Yusko, E. C.; Billeh, Y. N.; Macrae, M. X.; Yang, J.; Mayer, M. Applications of Biological Pores in Nanomedicine, Sensing, and Nanoelectronics. Curr. Opin. Biotechnol. 2010, 21 (4), 439-476.
(46) Cao, C.; Long, Y.-T. Biological Nanopores: Confined Spaces for Electrochemical Single-Molecule Analysis. Acc. Chem. Res. 2018, 51 (2), 331-341.
(47) Meng, F.-N.; Li, Z.-Y.; Ying, Y.-L.; Liu, S.-C.; Zhang, J.; Long, Y.-T. Structural Stability of the Photo-Responsive DNA Duplexes Containing One Azobenzene via a Confined Pore. Chem. Commun. 2017, 53 (68), 9462-9465.
(48) Wloka, C.; Mutter, N. L.; Soskine, M.; Maglia, G. Alpha-Helical Fragaceatoxin C Nanopore Engineered for Double-Stranded and Single-Stranded Nucleic Acid Analysis. Angew. Chem. Int. Ed. Engl. 2016, 55 (40), 12494-12498.
(49) Huang, G.; Voet, A.; Maglia, G. FraC Nanopores with Adjustable Diameter Identify the Mass of Opposite-Charge Peptides with 44 Dalton Resolution. Nat. Commun. 2019, 10 (1), 835.
(50) Huang, G.; Willems, K.; Soskine, M.; Wloka, C.; Maglia, G. Electro-Osmotic Capture and Ionic Discrimination of Peptide and Protein Biomarkers with FraC Nanopores. Nat. Commun. 2017, 8 (1), 935.
(51) Bayley, H. Nanopore Sequencing: From Imagination to Reality. Clin. Chem. 2015, 61 (1), 25-31.
(52) Quick, J.; Loman, N. J.; Duraffour, S.; Simpson, J. T.; Severi, E.; Cowley, L.; Bore, J. A.; Koundouno, R.; Dudas, G.; Mikhail, A.; et al. Real-Time, Portable Genome Sequencing for Ebola Surveillance. Nature 2016, 530 (7589), 228-232.
(53) Anderluh, G.; Barlic, A.; Potrich, C.; Macek, P.; Menestrina, G. Lysine 77 Is a Key Residue in Aggregation of Equinatoxin II, a Pore-Forming Toxin from Sea Anemone Actinia Equina. J. Membr. Biol. 2000, 173 (1), 47-55.
(54) Hong, Q.; Gutierrez-Aguirre, I.; Barlic, A.; Malovrh, P.; Kristan, K.; Podlesek, Z.; Macek, P.; Turk, D.; Gonzalez-Manas, J. M.; Lakey, J. H.; et al. Two-Step Membrane Binding by Equinatoxin II, a Pore-Forming Toxin from the Sea Anemone, Involves an Exposed Aromatic Cluster and a Flexible Helix. J. Biol. Chem. 2002, 277 (44), 41916-41924.
(55) Tanaka, K.; Caaveiro, J. M. M.; Morante, K.; González-Manãs, J. M.; Tsumoto, K. Structural Basis for Self-Assembly of a Cytolytic Pore Lined by Protein and Lipid. Nat. Commun. 2015, 6, 6337.
(56) Bakrac, B.; Gutiérrez-Aguirre, I.; Podlesek, Z.; Sonnen, A. F.-P.; Gilbert, R. J. C.; Macek, P.; Lakey, J. H.; Anderluh, G. Molecular Determinants of Sphingomyelin Specificity of a Eukaryotic Pore-Forming Toxin. J. Biol. Chem. 2008, 283 (27), 18665-18677.
(57) Szymański, W.; Wu, B.; Poloni, C.; Janssen, D. B.; Feringa, B. L. Azobenzene Photoswitches for Staudinger-Bertozzi Ligation. Angew. Chemie - Int. Ed. 2013, 52 (7), 2068-2072.
(58) Kalka, K.; Merk, H.; Mukhtar, H. Photodynamic Therapy in Dermatology. J. Am. Acad. Dermatol. 2000, 42 (3), 389-413; quiz 414-6.

## Claims

1. A modified Fragaceatoxin C (FraC) monomer capable of forming a photo-controlled FraC nanopore, the FraC monomer comprising a photoswitchable moiety covalently attached to one or more of the positions corresponding to K77, W112, E134, Y138, and S166 in the sequence of FraC from *Actina fragacea* (GenBank accession number FM958450).

2. Modified FraC monomer according to claim 1 that is capable of forming a FraC nanopore that is activated upon irradiation, the FraC monomer comprising a photoswitchable moiety covalently attached to one or more of the positions W112, E134 and Y138, preferably Y138.

3. Modified FraC monomer according to claim 1 that is capable of forming a FraC nanopore that is deactivated upon irradiation, the FraC monomer comprising a photoswitchable moiety covalently attached to position K77 and/or S166.

4. Modified FraC monomer according to any one of the preceding claims, being a mutant FraC monomer wherein a photoswitchable moiety is attached to a natural or unnatural amino acid residue introduced at one or more of the positions K77, W112, E134, Y138, and S166.

5. Modified mutant FraC monomer according to claim 4, wherein a photoswitchable moiety is attached to a Cysteine residue introduced at one or more of the positions K77, W112, E134, Y138, and S166.

6. Modified FraC monomer according to any one of claims 1-5, wherein said photoswitchable moiety is an azobenzene- or spiropyrane-based photoswitch.

7. Modified FraC monomer according to claim 6, wherein said azobenzene-based photoswitch comprises at the *para*-position a negatively or positively charged water-solubilizing substituent, preferably selected from a sulfonate group and a quaternary ammonium group.

8. A mutant FraC polypeptide comprising a natural or unnatural amino acid substitution at position K77, W112, E134, Y138 and/or S166, preferably comprising a Cys-residue at position K77, W112, E134 Y138 and/or S166.

9. A nucleic acid molecule encoding a mutant FraC polypeptide according to claim 8.

10. An expression vector comprising a nucleic acid molecule according to claim 9.

11. A host cell comprising an expression vector according to claim 10.

12. A photo-controlled FraC nanopore comprising a modified FraC monomer according to any one of claims 1-7.

13. A system comprising a photo-controlled FraC nanopore according to claim 12, wherein the nanopore is assembled in a (planar) lipid bilayer, preferably wherein the lipid bilayer comprises or consists of phosphatidylcholine (PC), preferably 1,2-diphytanoyl-*sn*-glycero-3-phosphocholine.

14. A photo-controlled FraC nanopore according to claim 13 for use as medicament, preferably as cytotoxic agent.

15. A pharmaceutical composition comprising a modified FraC monomer according to claim 2 and those depending thereon, and a pharmaceutically acceptable carrier, vehicle or diluent.

16. Photo-controlled FraC nanopore according to claim 12 or a pharmaceutical composition according to claim 15 for use in the treatment of cancer.

17. A photo-controlled FraC nanopore or system according to claim 12 or 13, for use as (bio)molecule sensor, preferably as a single-molecule sensor.

18. An azobenzene-based photoswitch compound that is responsive to visible light, the compound having the formula I 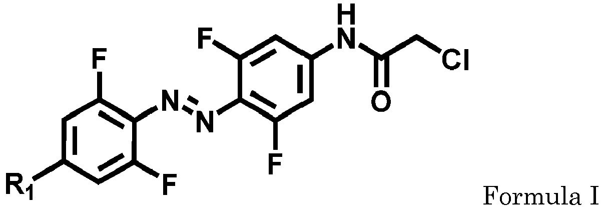 wherein R₁ is a charged group conferring water-solubility.
